# EUROPÄISCHE PATENTSCHRIFT

(11) **EP 3 238 780 B1**
(45) Veröffentlichungstag und Bekanntmachung des Hinweises auf die Patenterteilung: **25.09.2019**
(21) Anmeldenummer: 17187456.3
(22) Anmeldetag: 23.08.2017
(51) Int. Cl.: A61N 5/10

(54) **VERFAHREN ZUR UNTERSTÜTZUNG EINER PLANUNG EINER BESTRAHLUNG EINES PATIENTEN**
METHOD FOR PLANNING THE IRRADIATION OF A PATIENT
PROCÉDÉ POUR LA PLANIFICATION D'IRRADIATION D'UN PATIENT

(30) Priorität: 07.10.2016 DE 102016219496
(43) Veröffentlichungstag der Anmeldung: 01.11.2017
(73) Patentinhaber: Siemens Healthcare GmbH, 91052 Erlangen (DE)
(72) Erfinder: Nioutsikou, Elena, 91056 Erlangen (DE)

(56) Entgegenhaltungen:
- WO-A1-2015/171056
- DE-A1-102006 021 771
- US-A1- 2015 367 145
- JASON A. DOWLING ET AL: "An Atlas-Based Electron Density Mapping Method for Magnetic Resonance Imaging (MRI)-Alone Treatment Planning and Adaptive MRI-Based Prostate Radiation Therapy", INTERNATIONAL JOURNAL OF RADIATION ONCOLOGYBIOLOGYPHYSICS, Bd. 83, Nr. 1, 1. Mai 2012 (2012-05-01), Seiten e5-e11, XP055106054, ISSN: 0360-3016, DOI: 10.1016/j.ijrobp.2011.11.056
- A. TORRADO-CARVAJAL ET AL: "Fast pseudo-CT synthesis from MRI T1-weighted images using a patch-based approach", PROCEEDINGS OPTICAL DIAGNOSTICS OF LIVING CELLS II, Bd. 9681, 22. Dezember 2015 (2015-12-22), Seite 968106, XP055448905, US ISSN: 0277-786X, DOI: 10.1117/12.2210963 ISBN: 978-1-5106-1324-9
- A. TORRADO-CARVAJAL ET AL: "Fast Patch-Based Pseudo-CT Synthesis from T1-Weighted MR Images for PET/MR Attenuation Correction in Brain Studies", THE JOURNAL OF NUCLEAR MEDICINE, Bd. 57, Nr. 1, 22. Oktober 2015 (2015-10-22), Seiten 136-143, XP055448909, US ISSN: 0161-5505, DOI: 10.2967/jnumed.115.156299

## Beschreibung

Die Erfindung betrifft ein Verfahren zur Unterstützung einer Planung einer Bestrahlung eines Patienten, eine Recheneinheit und ein Computerprogrammprodukt.

Bei einer Strahlentherapie wird ein Zielvolumen, beispielsweise ein Tumor, eines Patienten mit ionisierender Strahlung bestrahlt. Hierbei ist eine externe Strahlentherapie, welche eine Bestrahlung eines Körpers des Patienten von außerhalb des Körpers umfasst, bekannt. Ebenfalls ist eine interne Strahlentherapie, auch Brachytherapie genannt, bekannt. Bei einer Brachytherapie werden Strahlenquellen, welche radioaktive Substanzen umfassen, in einen Körper des Patienten eingebracht, um lokal im Körper des Patienten das Tumorgewebe im Zielvolumen zu schädigen oder zu vernichten.

Grundsätzlich ist es eine Herausforderung bei der Bestrahlung des Patienten sicher zu stellen, dass eine derart ausreichende Strahlendosis einem Zielvolumen zugeführt wird, so dass das im Zielvolumen enthaltene Tumorgewebe zerstört wird. Gleichzeitig sollen das Zielvolumen umgebene Risikoorgane möglichst geschont werden. Damit ist eine hohe Genauigkeit bei der Planung der Bestrahlung von hoher Bedeutung.

Es ist bekannt eine Strahlentherapie bzw. eine Bestrahlung eines Patienten mittels einer Bildgebung zu planen und/oder zu überwachen. Hierzu wird üblicherweise ein Bestrahlungsplan mit Hilfe von medizinischen Bilddaten des Patienten erstellt, die mit einem dreidimensionalen bildgebenden Verfahren erstellt wurden. Üblicherweise werden hierfür Computertomographie-Bilddaten (CT-Bilddaten) eingesetzt. Anhand der Computertomographie-Bilddaten kann einerseits das Zielvolumen der Bestrahlung festgelegt werden, und andererseits umliegende Risikoorgane lokalisiert werden. Darüber hinaus bilden die Intensitätswerte der Bildvoxel der Bilddaten (gemessen in sogenannten "Hounsfield Units") in guter Näherung eine Elektronendichte am korrespondierenden Ort im Körper des Patienten ab, da die Intensitätswerte der Bildvoxel auf einer Absorption der Röntgenstrahlung an den zugehörigen Orten beruhen. Derart können die Computertomographie-Bilddaten besonders einfach für die Bestrahlungsplanung in eine Elektronendichtekarte umgerechnet werden. Da bei einer Bestrahlung die Intensität der Wechselwirkung der Strahlung mit der Elektronendichte im Körper korreliert, kann aus den Computertomographie-Bilddaten vergleichsweise einfach die Schwächung der Strahlung beim Durchtritt durch den Körper errechnet werden. Aufgrund dieser Eigenschaft wurden Computertomographie-Bilddaten bisher bei der Erstellung einer Bestrahlungsplanung bevorzugt eingesetzt. Computertomographie-Bilddaten weisen weiterhin nur eine geringe geometrische Verzerrung auf und ermöglichen somit eine geeignete Definition einer Referenzgeometrie für die Planung der Bestrahlung und das Durchführen der Bestrahlung.

Es besteht allerdings die Forderung, andere bildgebende Verfahren in der Planung der Bestrahlung einzusetzen, die einen besseren Weichteilkontrast aufweisen, um eine verbesserte Identifikation des Tumorgewebes im Zielvolumen und/oder der Risikoorgane zu ermöglichen. Ein solches Bildgebungsverfahren, das der Forderung nach einem besseren Weichteilkontrast gerecht wird, ist die Magnetresonanz-Bildgebung (MR-Bildgebung) mittels eines Magnetresonanzgeräts. Bei einer derartigen Bildgebung hängt der Kontrast von der Verteilung der Spindichte, der Wechselwirkung der Spins untereinander und/oder mit ihrer Umgebung ab. Hierdurch kann ein Weichteilkontrast erreicht werden, der deutlich über dem mit einem Computertomographiegerät erreichbaren Kontrast liegt.

In einem Magnetresonanzgerät, auch Magnetresonanztomographiesystem genannt, wird üblicherweise der zu untersuchende Körper einer Untersuchungsperson, insbesondere eines Patienten, mit Hilfe eines Hauptmagneten einem relativ hohen Hauptmagnetfeld, beispielsweise von 1,5 oder 3 oder 7 Tesla, ausgesetzt. Zusätzlich werden mit Hilfe einer Gradientenspuleneinheit Gradientenpulse ausgespielt. Über eine Hochfrequenzantenneneinheit werden dann mittels geeigneter Antenneneinrichtungen Hochfrequenz-Pulse, insbesondere Anregungspulse, ausgesendet, was dazu führt, dass die Kernspins bestimmter, durch diese Hochfrequenz-Pulse resonant angeregter Atome um einen definierten Flipwinkel gegenüber den Magnetfeldlinien des Hauptmagnetfelds verkippt werden. Bei der Relaxation der Kernspins werden Hochfrequenz-Signale, so genannte Magnetresonanz-Signale, abgestrahlt, die mittels geeigneter Hochfrequenzantennen empfangen und dann weiterverarbeitet werden. Aus den so akquirierten Rohdaten können schließlich die gewünschten Bilddaten rekonstruiert werden.

Bekannt ist derart ein kombinierter Einsatz von Computertomographie-Bildgebung und Magnetresonanz-Bildgebung zur Planung einer Bestrahlung. Für die Planung der Bestrahlung werden dann typischerweise die akquirierten Computertomographie-Bilddaten und Magnetresonanz-Bilddaten durch Bildregistrierung überlagert. Hierbei kann jedoch das Problem auftreten, dass eine nicht vollständig korrekt durchgeführte Bildregistrierung systematische Fehler in die Planung der Bestrahlung einführen kann. Auch müssen bei dem kombinierten Einsatz von Computertomographie-Bildgebung und Magnetresonanz-Bildgebung die Bilddaten mit den beiden Modalitäten in einem kurzen Abstand zueinander akquiriert werden, damit Veränderungen in der Anatomie des Patienten, beispielsweise aufgrund einer unterschiedlichen Füllung der Blase des Patienten, zwischen den Akquisitionen vermieden werden können. Dies kann zu operativen Herausforderungen, erhöhten Kosten und zu einem verringerten Patientenkomfort führen, da der Patient mehrere Untersuchungen erdulden muss.

Aus der Fig. 4 der DE 10 2006 021771 A1 ist ein Verfahren zum Erstellen einer Bestrahlungsplanung mit einem CT-Abbild und einem MR-Abbild bekannt, wobei ein CT-Abbild des Patienten zunächst erstellt wird und mit einem zuvor aufgenommenen MR-Abbild des Patienten registriert wird.

Aus den folgenden Schriften sind Methoden zur Erstellung eines Pseudo-CT Bilddatensatzes aus einem Magnetresonanz-Bilddatensatz bekannt:
- US 2015/367145 A1
- WO 2015/171056 A1
- JASON A. DOWLING ET AL: "An Atlas-Based Electron Density Mapping Method for Magnetic Resonance Imaging (MRI)-Alone Treatment Planning and Adaptive MRI-Based Prostate Radiation Therapy", INTERNATIONAL JOURNAL OF RADIATION ONCOLOGYBIOLOGYPHYSICS, Bd. 83, Nr. 1, 1. Mai 2012 (2012-05-01), Seiten e5-e11,
- A. TORRADO-CARVAJAL ET AL: "Fast pseudo-CT synthesis from MRI T1-weighted images using a patch-based approach", PROCEEDINGS OPTICAL DIAGNOSTICS OF LIVING CELLS II, Bd. 9681, 22. Dezember 2015 (2015-12-22), Seite 968106A. TORRADO-CARVAJAL ET AL: "Fast Patch-Based Pseudo- CT Synthesis from T1-Weighted MR Images for PET/MR Attenuation Correction in Brain Studies", THE JOURNAL OF NUCLEAR MEDICINE, Bd. 57, Nr. 1, 22. Oktober 2015 (2015-10-22), Seiten 136-143,

Der Erfindung liegt die Aufgabe zu Grunde, eine verbesserte Unterstützung der Planung der Bestrahlung des Patienten zu ermöglichen. Die Aufgabe wird durch die Merkmale der unabhängigen Ansprüche gelöst. Vorteilhafte Ausgestaltungen sind in den Unteransprüchen beschrieben.

Das erfindungsgemäße Verfahren zur Unterstützung einer Planung einer Bestrahlung eines Patienten, umfasst folgende Verfahrensschritte:
- Erfassen von Computertomographie-Bilddaten des Patienten, welche von dem Patienten mittels eines Computertomographiegeräts akquiriert worden sind,
- Zuordnen von Magnetresonanz-Bilddaten zu den Computertomographie-Bilddaten unter Verwendung der Computertomographie-Bilddaten, wobei die Magnetresonanz-Bilddaten von zumindest einem von dem Patienten verschiedenen Untersuchungsobjekt mittels eines Magnetresonanzgeräts akquiriert worden sind, und
- Bereitstellen der Computertomographie-Bilddaten zusammen mit den den Computertomographie-Bilddaten zugeordneten Magnetresonanz-Bilddaten zur Unterstützung der Planung der Bestrahlung des Patienten.

Das Erfassen der Computertomographie-Bilddaten kann direkt die Akquisition der Computertomographie-Bilddaten mittels des Computertomographiegeräts umfassen. Alternativ kann das Erfassen der Computertomographie-Bilddaten ein Laden der bereits vom Patienten akquirierten Computertomographie-Bilddaten aus einer Datenbank umfassen. In allen Fällen umfassten die Computertomographie-Bilddaten insbesondere anatomische Bildinformationen desjenigen Patienten, welcher bestrahlt werden soll.

Das Zuordnen der Magnetresonanz-Bilddaten zu den Computertomographie-Bilddaten kann mittels eines Algorithmus erfolgen, welcher als Eingangsparameter die Magnetresonanz-Bilddaten und die Computertomographie-Bilddaten und als Ausgangsparameter den Computertomographie-Bilddaten zugeordnete Magnetresonanz-Bilddaten aufweist. Der Algorithmus setzt dabei die Computertomographie-Bilddaten selbst ein, damit die Zuordnung der Magnetresonanz-Bilddaten zu den Computertomographie-Bilddaten möglich ist. Es ist dabei eine direkte Registrierung der Magnetresonanz-Bilddaten auf die Computertomographie-Bilddaten denkbar. Da diese jedoch technisch oft schwer zu realisieren ist, wird vorteilhafterweise ein atlasbasiertes Verfahren zum Zuordnen der Magnetresonanz-Bilddaten zu den Computertomographie-Bilddaten eingesetzt. Möglichkeiten für das atlas-basierte Verfahren werden in einem der folgenden Abschnitte noch genauer beschrieben.

Die Magnetresonanz-Bilddaten werden für die Zuordnung zu den Computertomographie-Bilddaten insbesondere aus einer Datenbank geladen. Vorteilhafterweise kann dabei, wie in einem der folgenden Abschnitte noch genauer beschrieben, die Datenbank einen Atlas, welcher die Magnetresonanz-Bilddaten zusammen mit korrespondierenden Atlas-Computertomographie-Bilddaten umfasst, umfassen. Die Magnetresonanz-Bilddaten sind insbesondere ausschließlich von dem zumindest einen von dem Patienten verschiedenen Untersuchungsobjekt mittels eines Magnetresonanzgeräts akquiriert worden. Dies bedeutet, dass insbesondere für die Erstellung der Magnetresonanz-Bilddaten der Patient nicht mittels des Magnetresonanzgeräts untersucht werden muss.

Vorteilhafterweise sind die Magnetresonanz-Bilddaten von mehreren von dem Patienten verschiedenen Untersuchungsobjekten mittels des Magnetresonanzgeräts akquiriert worden. So können die Magnetresonanz-Bilddaten beispielsweise durchschnittliche, vorteilhafterweise allgemeingültige, anatomische Merkmale, welche auf den Akquisitionen der mehreren von dem Patienten verschiedenen Untersuchungsobjekten basieren, umfassen. Selbstverständlich können die Magnetresonanz-Bilddaten auch mit mehreren unterschiedlichen Magnetresonanzgeräten akquiriert worden sein, wenn sie von mehreren von dem Patienten verschiedenen Untersuchungsobjekten akquiriert worden sind.

Das Ergebnis der Zuordnung der Magnetresonanz-Bilddaten zu den Computertomographie-Bilddaten sind insbesondere die den Computertomographie-Bilddaten zugeordnete Magnetresonanz-Bilddaten. Diese den Computertomographie-Bilddaten zugeordnete Magnetresonanz-Bilddaten weisen insbesondere gegenüber den ursprünglichen Magnetresonanz-Bilddaten einen geänderten Bildinhalt auf. Wenn beispielsweise das Zuordnen der Magnetresonanz-Bilddaten zu den Computertomographie-Bilddaten eine räumliche Transformation der Magnetresonanz-Bilddaten umfasst, dann sind die den Computertomographie-Bilddaten zugeordneten Magnetresonanz-Bilddaten gegenüber den ursprünglichen Magnetresonanz-Bilddaten insbesondere räumlich transformiert ausgebildet. Die den Computertomographie-Bilddaten zugeordneten Magnetresonanz-Bilddaten sind insbesondere räumlich an die Computertomographie-Bilddaten angepasst, befinden sich also vorteilhafterweise möglichst in räumlicher Übereinstimmung zu den Computertomographie-Bilddaten. Die den Computertomographie-Bilddaten zugeordneten Magnetresonanz-Bilddaten bilden demnach insbesondere eine Anatomie ab, welche möglichst auf Grundlage der vom Patient akquirierten Computertomographie-Bilddaten an die Anatomie des Patienten angepasst ist. Die den Computertomographie-Bilddaten zugeordneten Magnetresonanz-Bilddaten sind demnach vorteilhafterweise derart ausgebildet, dass bei einem Übereinanderlegen der den Computertomographie-Bilddaten zugeordneten Magnetresonanz-Bilddaten und der Computertomographie-Bilddaten anatomische Strukturen des Patienten in beiden Bilddaten sich an den gleichen Stellen befinden.

Das Bereitstellen der Computertomographie-Bilddaten zusammen mit den den Computertomographie-Bilddaten zugeordneten Magnetresonanz-Bilddaten kann eine Anzeige der beiden Bilddaten für einen Benutzer auf einer Anzeigeeinheit umfassen. Die beiden Bilddaten können dabei gleichzeitig, beispielsweise nebeneinander, auf der Anzeigeeinheit dargestellt werden. Es ist auch eine fusionierte Darstellung der Computertomographie-Bilddaten und der den Computertomographie-Bilddaten zugeordneten Magnetresonanz-Bilddaten auf der Anzeigeeinheit denkbar. Alternativ oder zusätzlich kann das Bereitstellen ein Abspeichern der beiden Bilddaten in der Datenbank umfassen. Alternativ oder zusätzlich kann das Bereitstellen ein Übertragen der beiden Bilddaten an ein Weiterverarbeitungssystem, beispielsweise ein Bildverarbeitungssystem, umfassen, wobei das weitere Rechensystem die beiden Bilddaten dann weiter verarbeiten kann.

Die Computertomographie-Bilddaten werden insbesondere zusammen mit den den Computertomographie-Bilddaten zugeordneten Magnetresonanz-Bilddaten derart bereitgestellt, dass beide Bilddaten zur Unterstützung der Planung der Bestrahlung des Patienten beitragen können. Dabei werden, wie in einem der folgenden Abschnitte noch genauer beschrieben, zur Unterstützung der Planung der Bestrahlung die Computertomographie-Bilddaten zu einem anderen Zweck bereitgestellt werden als die den Computertomographie-Bilddaten zugeordneten Magnetresonanz-Bilddaten. An das Bereitstellen der Computertomographie-Bilddaten zusammen mit den den Computertomographie-Bilddaten zugeordneten Magnetresonanz-Bilddaten kann sich somit ein Planungsschritt anschließen, in welchem zumindest ein Teil der Planung der Bestrahlung des Patienten unter Verwendung der bereitgestellten Computertomographie-Bilddaten zusammen mit den den Computertomographie-Bilddaten zugeordneten Magnetresonanz-Bilddaten durchgeführt wird.

Das vorgeschlagene Vorgehen kann somit insbesondere als Umkehrung der bekannten ausschließlich Magnetresonanz-basierten Bestrahlungsplanung ("MR-only RT Planning", MRORTP) angesehen werden. Diese ausschließlich Magnetresonanz-basierten Bestrahlungsplanung würde vorsehen, Computertomographie-Bildgebung für geeignete klinische Anwendungsfälle aus dem Planungsprozess vollständig zu eliminieren. Für die ausschließlich Magnetresonanz-basierte Bestrahlungsplanung muss in vielen Fällen dennoch auf Informationen aus Computertomographie-Bildgebung zurückgegriffen werden, da eine für die Dosisberechnung benötigte Elektronendichtekarte erstellt werden muss. Die Erstellung der für die Dosisberechnung benötigten Elektronendichtekarte ist typischer ausschließlich mittels der akquirierten Magnetresonanz-Bilddaten schwer möglich. Daher kann beispielsweise auf ein bekanntes atlasbasiertes Verfahren zurückgegriffen werden, bei dem auf Informationen aus in einem Atlas hinterlegten Computertomographie-Bilddaten zugegriffen wird, um die Elektronendichtekarte für die ausschließlich Magnetresonanz-basierten Bestrahlungsplanung zu erstellen.

Hierbei sind atlas-basierte Registrierungsverfahren zum Erstellen der Elektronendichtekarte für die ausschließlich Magnetresonanz-basierten Bestrahlungsplanung bekannt, wobei beispielsweise auf die Schrift von Dowling et al., "An Atlas-Based Electron Density Mapping Method for Magnetic Resonance Imaging (MRI)-Alone Treatment Planning and Adaptive MRI-Based Prostate Radiation Therapy", Int J Radiation Oncol Biol Phys, 83(1), e5-e11, 2012 verwiesen sind. Alternativ sind atlas-basierte Verfahren, welche eine patch-basierten Methode einsetzen, bekannt. Hierbei wird beispielsweise auf die Schrift von Torrado-Carvajal et al., "Fast Patch-Based Pseudo-CT Synthesis from T1-Weighted MR Images for PET/MR Attenuation Correction in Brain Studies", J Nucl Med, 57, 136-143, 2016 verwiesen, wobei das in dieser Schrift beschriebene Verfahren problemlos statt auf PET/MR Schwächungskorrektur auch auf die Berechnung der Elektronendichte angewendet werden kann.

Im Gegensatz zu den in den zwei vorhergehenden Absätzen beschriebenen bekannten Verfahren, können mittels des vorgeschlagene Verfahrens vorteilhafterweise zusätzlich Informationen aus Magnetresonanz-Bildgebung für eine Planung der Bestrahlung des Patienten gewonnen werden, ohne dass eine Akquisition der Magnetresonanz-Bilddaten des Patienten nötig ist. Das beschriebene Vorgehen sieht also insbesondere eine Planung der Bestrahlung des Patienten vor, für welche ausschließlich Computertomographie-Bilddaten des Patienten akquiriert werden müssen. Dennoch können mittels des beschriebenen Vorgehens vorteilhafterweise auch Magnetresonanz-Bilddaten zur Unterstützung der Planung der Bestrahlung hinzugezogen werden, ohne dass eine Akquisition der Magnetresonanz-Bilddaten von dem Patienten nötig ist. Die Tatsache, dass die Akquisition der Magnetresonanz-Bilddaten für die Planung der Bestrahlung des Patienten entfallen kann, kann vorteilhafterweise zu erheblichen Vereinfachungen im Arbeitsablauf und/oder zu einer Einsparung von Kosten und/oder zu einer Erhöhung des Patientenkomforts führen.

Die den Computertomographie-Bilddaten zugeordneten Magnetresonanz-Bilddaten können dabei als pseudo-MR Bilddaten angesehen werden, da sie nicht direkt vom Patienten mittels des Magnetresonanzgeräts akquiriert worden sind. Die den Computertomographie-Bilddaten zugeordneten Magnetresonanz-Bilddaten können für die Planung der Bestrahlung des Patienten besonders vorteilhaft einen gegenüber den Computertomographie-Bilddaten erhöhten Weichteilkontrast aufweisen. Während die Computertomographie-Bilddaten typischerweise nur Grenzen eines Organs aufweisen, können die den Computertomographie-Bilddaten zugeordneten Magnetresonanz-Bilddaten besonders vorteilhaft spezifischere Informationen, beispielsweise über eine genaue Ausdehnung des Tumorgewebes im Zielvolumen und/oder über eine Lokalisation der Risikoorgane und/oder über eine Aktivität des Tumorgewebes, bereitstellen. Gleichzeitig können sich in den den Computertomographie-Bilddaten zugeordneten Magnetresonanz-Bilddaten Tumorgewebe und/oder Risikoorgane mit einem höheren Kontrast von umliegenden Gewebe als in den Computertomographie-Bilddaten abheben. So können für die Planung der Bestrahlung des Patienten beispielsweise besonders genau das Tumorgewebe und/oder die Risikoorgane unter Verwendung der den Computertomographie-Bilddaten zugeordneten Magnetresonanz-Bilddaten identifiziert werden. Selbstverständlich können die Computertomographie-Bilddaten selbst weiterhin noch sinnvoll für die Planung der Bestrahlung des Patienten eingesetzt werden, beispielsweise zur Abgrenzung der Knochen und/oder zum Berechnen der Elektronendichtekarte.

Eine Ausführungsform sieht vor, dass zur Unterstützung der Planung der Bestrahlung die Computertomographie-Bilddaten zu einem anderen Zweck bereitgestellt werden als die den Computertomographie-Bilddaten zugeordneten Magnetresonanz-Bilddaten.

Besonders vorteilhaft ermöglicht das beschriebene Vorgehen nämlich Tatsache, dass sowohl die Computertomographie-Bilddaten, also auch die den Computertomographie-Bilddaten zugeordneten Magnetresonanz-Bilddaten zur Unterstützung der Planung der Bestrahlung des Patienten zur Verfügung stehen. Da die den Computertomographie-Bilddaten zugeordneten Magnetresonanz-Bilddaten vorteilhafterweise Bildinformationen aufweisen, welche die Bildinformationen der Computertomographie-Bilddaten ergänzen können, können bei der Unterstützung der Planung der Bestrahlung die den Computertomographie-Bilddaten zugeordneten Magnetresonanz-Bilddaten besonders vorteilhaft für bei anderen Teilaufgaben der Planung der Bestrahlung unterstützen als die Computertomographie-Bilddaten. So können ein erster Satz von Teilaufgaben der Planung der Bestrahlung unter Verwendung der Computertomographie-Bilddaten und ein zweiter Satz von Teilaufgaben der Planung der Bestrahlung unter Verwendung der den Computertomographie-Bilddaten zugeordneten Magnetresonanz-Bilddaten durchgeführt werden. Der erste Satz und der zweite Satz der Teilaufgabe sind dabei vorteilhafterweise zumindest teilweise disjunkt.

Eine Ausführungsform sieht vor, dass zur Unterstützung der Planung der Bestrahlung die Computertomographie-Bilddaten zu zumindest einem der folgenden Zwecke bereitgestellt werden:
- eine Verifizierung einer korrekten Positionierung des Patienten für die Bestrahlung und
- eine Berechnung einer Strahlendosis während der Planung der Bestrahlung.

Derart wird die Verifizierung der korrekten Positionierung des Patienten für die Bestrahlung und/oder die Berechnung der Strahlendosis während der Planung der Bestrahlung, insbesondere ausschließlich, unter Verwendung der Computertomographie-Bilddaten durchgeführt.

Die Verifizierung der korrekten Positionierung des Patienten für die Bestrahlung wird dabei insbesondere unter Verwendung der Computertomographie-Bilddaten und weiteren Bilddaten durchgeführt. Die weiteren Bilddaten werden insbesondere dann aufgenommen, wenn der Patient bereits für die Bestrahlung positioniert worden ist. Die weiteren Bilddaten können dabei beispielsweise optische Kamerabilddaten und/oder Röntgenbilddaten und/oder mittels einer Portalbildgebungsquelle des Strahlentherapiegeräts akquirierte Bilddaten umfassen. Für die Verifizierung der korrekten Positionierung des Patienten können dann die Computertomographie-Bilddaten vorteilhafterweise mit den weiteren Bilddaten verglichen werden. Mögliche Verfahren zur Verifizierung der korrekten Positionierung des Patienten sind dabei dem Fachmann bekannt, so dass hier nicht genauer auf diese eingegangen werden soll. Die Computertomographie-Bilddaten können besonders vorteilhaft zur Verifizierung der korrekten Positionierung des Patienten für die Bestrahlung eingesetzt werden, da sie nur eine sehr geringe bzw. praktisch keine geometrische Verzerrung aufweisen und somit eine zuverlässige Referenzgeometrie bestimmen können. So können die Computertomographie-Bilddaten positionsgetreuer als Magnetresonanz-Bilddaten sein.

Für Berechnung der Strahlendosis während der Planung der Bestrahlung wird aus den Computertomographie-Bilddaten typischerweise eine Elektronendichtekarte aus den Computertomographie-Bilddaten verwendet. Da die Computertomographie-Bilddaten insbesondere direkt eine ortsaufgelöste Verteilung der Elektronendichte im Patient wiederspiegeln, kann die Berechnung der Elektronendichtekarte aus den Computertomographie-Bilddaten besonders einfach, insbesondere mittels eines dem Fachmann bekannten Verfahrens, erfolgen. Die aus den Computertomographie-Bilddaten berechnete Elektronendichtekarte kann dann, wie es dem Fachmann geläufig ist, für die Berechnung der Strahlendosis im Zielvolumen bzw. in Risikoorganen des Patienten anhand von eingestellten Bestrahlungsparametern verwendet werden.

Eine Ausführungsform sieht vor, dass zur Unterstützung der Planung der Bestrahlung die den Computertomographie-Bilddaten zugeordneten Magnetresonanz-Bilddaten zu zumindest einem der folgenden Zwecke bereitgestellt werden:
- eine Konturierung einer Organstruktur des Patienten für die Planung der Bestrahlung und
- eine automatische Segmentierung einer Organstruktur des Patienten für die Planung der Bestrahlung.

Derart wird die Konturierung der Organstruktur des Patienten für die Planung der Bestrahlung und/oder die automatische Segmentierung der Organstruktur des Patienten für die Planung der Bestrahlung, insbesondere ausschließlich, unter Verwendung der den Computertomographie-Bilddaten zugeordneten Magnetresonanz-Bilddaten durchgeführt. Denkbar ist auch, dass für die Konturierung und/oder Segmentierung der Organstruktur sowohl die Computertomographie-Bilddaten als auch die den Computertomographie-Bilddaten zugeordneten Magnetresonanz-Bilddaten verwendet werden. Gerade für die Konturierung kann beispielsweise eine fusionierte Anzeige der Computertomographie-Bilddaten und der den Computertomographie-Bilddaten zugeordneten Magnetresonanz-Bilddaten vorteilhaft sein.

Die Organstruktur kann beispielsweise ein bei der Bestrahlung zu bestrahlendes Zielorgan bzw. ein im Zielvolumen lokalisiertes Tumorgewebe sein. Alternativ oder zusätzlich kann die Organstruktur ein Risikoorgan sein, welches bei der Bestrahlung geschont werden soll. Die Konturierung und/oder automatische Segmentierung der Organstruktur ist somit typischerweise grundlegend für die Planung der Bestrahlung.

Bei der Konturierung und/oder automatischen Segmentierung der Organstruktur können die den Computertomographie-Bilddaten zugeordneten Magnetresonanz-Bilddaten gegenüber den Computertomographie-Bilddaten wertvolle Zusatzinformationen liefern. Wie bereits erwähnt, spielt hierbei insbesondere der ausgeprägte Weichteilkontrast der den Computertomographie-Bilddaten zugeordneten Magnetresonanz-Bilddaten eine große Rolle. Da die Computertomographie-Bilddaten und die den Computertomographie-Bilddaten zugeordneten Magnetresonanz-Bilddaten insbesondere räumlich in Übereinstimmung gebracht worden sind, können die Konturen bzw. die Segmentierung besonders einfach von den den Computertomographie-Bilddaten zugeordneten Magnetresonanz-Bilddaten auf die Computertomographie-Bilddaten für die weitere Planung der Bestrahlung, beispielsweise die Dosisberechnung unter Verwendung der aus den Computertomographie-Bilddaten berechneten Elektronendichtekarte, übertragen werden.

Eine Ausführungsform sieht vor, dass das Zuordnen der Magnetresonanz-Bilddaten zu den Computertomographie-Bilddaten mittels eines atlas-basierten Verfahrens erfolgt, welches folgende Schritte umfasst:
- Bereitstellen eines Atlas, welcher die Magnetresonanz-Bilddaten zusammen mit korrespondierenden Atlas-Computertomographie-Bilddaten des zumindest einem von dem Patienten verschiedenen Untersuchungsobjekts umfasst,
- Zuordnen der Atlas-Computertomographie-Bilddaten zu den Computertomographie-Bilddaten,
- Zuordnen der Magnetresonanz-Bilddaten zu den Computertomographie-Bilddaten unter Verwendung von Informationen, welche sich aus dem Zuordnen der Atlas-Computertomographie-Bilddaten zu den Computertomographie-Bilddaten ergeben.

Der Atlas ist insbesondere in einer Datenbank abgelegt bzw. stellt zumindest einen Teil einer Datenbank dar. Für das Zuordnen der Magnetresonanz-Bilddaten zu den Computertomographie-Bilddaten wird dann der Atlas insbesondere aus der Datenbank geladen.

Die Atlas-Computertomographie-Bilddaten sind insbesondere - wie die Magnetresonanz-Bilddaten - ausschließlich von dem zumindest einen von dem Patienten verschiedenen Untersuchungsobjekt mittels eines Computertomographiegeräts akquiriert worden. Dies bedeutet, dass insbesondere für die Erstellung der Atlas-Computertomographie-Bilddaten der Patient nicht mittels des Computertomographiegeräts untersucht werden muss. Bezüglich der Akquisition der Atlas-Computertomographie-Bilddaten von mehreren von dem Patienten verschiedenen Untersuchungsobjekten wird auf den entsprechenden Absatz der Beschreibung der Akquisition der Magnetresonanz-Bilddaten von mehreren von dem Patienten verschiedenen Untersuchungsobjekten verwiesen. Vorteilhafterweise werden die Atlas-Computertomographie-Bilddaten und die Magnetresonanz-Bilddaten von dem gleichen zumindest einem von dem Patienten verschiedenen Untersuchungsobjekt akquiriert. Es können allerdings auch unterschiedliche von dem Patienten verschiedene Untersuchungsobjekte in die Erstellung der Magnetresonanz-Bilddaten und der Atlas-Computertomographie-Bilddaten einfließen.

Im Atlas sind die Magnetresonanz-Bilddaten insbesondere in räumlicher Übereinstimmung zu den Atlas-Computertomographie-Bilddaten hinterlegt. Dies kann bedeuten, dass die Magnetresonanz-Bilddaten im Atlas registriert zu den Atlas-Computertomographie-Bilddaten hinterlegt sind. Insbesondere kann im Atlas zumindest ein zueinander registriertes Paar aus Magnetresonanz-Bilddaten und korrespondierenden Atlas-Computertomographie-Bilddaten hinterlegt sein. Die Magnetresonanz-Bilddaten und korrespondierenden Atlas-Computertomographie-Bilddaten können dabei einen gleichen anatomischen Bereich, beispielsweise einen Kopfbereich, einen Thoraxbereich oder einen Pelvisbereich, abbilden.

Für die Erstellung des Atlas werden die Magnetresonanz-Bilddaten und die Atlas-Computertomographie-Bilddaten vorteilhafterweise mit einem möglichst kurzen Zeitabstand, höchst vorteilhafterweise unmittelbar zeitlich aufeinanderfolgend, von dem zumindest einen von dem Patienten verschiedenen Untersuchungsobjekt aufgenommen. Derart kann eine besonders hohe anatomische Übereinstimmung zwischen den Magnetresonanz-Bilddaten und den Atlas-Computertomographie-Bilddaten vorliegen. Werden die Magnetresonanz-Bilddaten und die Atlas-Computertomographie-Bilddaten von mehreren von dem Patienten verschiedenen Untersuchungsobjekten akquiriert, so werden vorteilhafterweise gleiche Akquisitionsparameter, beispielsweise ein gleiches CT-Spektrum oder eine gleiche Magnetresonanz-Sequenz, für die Akquisition der Magnetresonanz-Bilddaten und der Atlas-Computertomographie-Bilddaten von den mehreren von dem Patienten verschiedenen Untersuchungsobjekten verwendet.

Die Atlas-Computertomographie-Bilddaten können besonders einfach zu den Computertomographie-Bilddaten des Patienten zugeordnet werden, da beide Bilddaten der gleichen Modalität angehören. Das Zuordnen der Atlas-Computertomographie-Bilddaten zu den Computertomographie-Bilddaten des Patienten umfasst dabei insbesondere eine räumliche Zuordnung, so dass die Atlas-Computertomographie-Bilddaten in räumliche Übereinstimmung zu den Computertomographie-Bilddaten des Patienten gebracht werden. Das Zuordnen der Atlas-Computertomographie-Bilddaten zu den erfassten Computertomographie-Bilddaten kann dabei mittels verschiedener Verfahren erfolgen, von denen zwei exemplarisch in den folgenden Ausführungsformen beschrieben werden.

Das Zuordnen der Atlas-Computertomographie-Bilddaten zu den Computertomographie-Bilddaten des Patienten kann eine vorteilhafte Grundlage für das Zuordnen der Magnetresonanz-Bilddaten zu den Computertomographie-Bilddaten des Patienten bieten. So können aus dem Zuordnen der Atlas-Computertomographie-Bilddaten zu den Computertomographie-Bilddaten Zuordnungsparameter, beispielsweise Registrierungsparameter wie ein bei der Registrierung erhaltenen Deformationsfeld, ermittelt werden. Diese Zuordnungsparameter können dann als Eingangsparameter in den Zuordnungsalgorithmus, welcher die Magnetresonanz-Bilddaten den Computertomographie-Bilddaten des Patienten zuordnet, eingehen.

Das direkte Zuordnen der Magnetresonanz-Bilddaten aus dem Atlas zu den Computertomographie-Bilddaten des Patienten, beispielsweise mittels einer Registrierung, kann dagegen schwierig sein, da beide Bilddaten unterschiedlichen Modalitäten angehören. Das vorgeschlagene atlas-basierte Verfahren, welches den Einsatz eines zweiteiligen Atlas, welcher zueinander korrespondierende Magnetresonanz-Bilddaten und Atlas-Computertomographie-Bilddaten enthält, bietet somit vorteilhafte Verbesserungen für das Zuordnen der Magnetresonanz-Bilddaten zu den Computertomographie-Bilddaten des Patienten.

Eine Ausführung sieht vor, dass das Zuordnen der Atlas-Computertomographie-Bilddaten zu den Computertomographie-Bilddaten eine Registrierung der Atlas-Computertomographie-Bilddaten auf die Computertomographie-Bilddaten umfasst, wobei das Zuordnen der Magnetresonanz-Bilddaten zu den Computertomographie-Bilddaten unter Verwendung eines bei der vorhergehenden Registrierung ermittelten Deformationsfelds erfolgt.

Die Registrierung der Atlas-Computertomographie-Bilddaten auf die Computertomographie-Bilddaten kann mit einer rigiden, affinen oder vorteilhafterweise mit einer nicht-rigiden Registrierungsmethode erfolgen. Ein Ergebnis der Registrierung ist dann insbesondere das Deformationsfeld, welches die räumliche Deformation der Atlas-Computertomographie-Bilddaten beschreibt, welche nötig ist, um die Atlas-Computertomographie-Bilddaten auf die Computertomographie-Bilddaten des Patienten abzubilden. Dieses Deformationsfeld kann dann besonders vorteilhaft für eine Deformation der Magnetresonanz-Bilddaten eingesetzt werden, um diese räumlich den Computertomographie-Bilddaten des Patienten zuzuordnen. Technische Einzelheiten zu atlas-basierten Registrierungsverfahren zum Erstellen einer Elektronendichtekarte für die ausschließlich Magnetresonanz-basierten Bestrahlungsplanung sind aus der bereits zitierten Schrift von Dowling et al. bekannt.

Eine Ausführung sieht vor, dass das Zuordnen der Atlas-Computertomographie-Bilddaten zu den Computertomographie-Bilddaten mit einer patch-basierten Methode erfolgt, wobei das Zuordnen der Magnetresonanz-Bilddaten zu den Computertomographie-Bilddaten unter Verwendung von Informationen erfolgt, welche bei der patch-basierten Methode gewonnen werden.

Für die patch-basierte Methode sind die Atlas-Computertomographie-Bilddaten vorteilhafterweise in mehrere Stücke, sogenannte "patches", aufgeteilt. Es kann dann eine lokale Ähnlichkeit der Stücke der Atlas-Computertomographie-Bilddaten zu räumlichen Bereichen der Computertomographie-Bilddaten des Patienten bestimmt werden. Basierend auf der bestimmten lokalen Ähnlichkeit können Wichtungsfaktoren berechnet werden, welche für eine entsprechende Anpassung der Magnetresonanz-Bilddaten auf die Computertomographie-Bilddaten des Patienten verwendet werden können. Technische Einzelheiten zu patch-basierten Verfahren zum Erstellen einer Schwächungskarte basierend auf Magnetresonanz-Bilddaten sind aus der bereits zitierten Schrift von Torrado-Carvajal et al. bekannt, wobei das in dieser Schrift beschriebene Verfahren statt auf PET/MR Schwächungskorrektur auch auf die Berechnung der Elektronendichte angewendet werden kann.

Eine Ausführungsform sieht vor, dass die Atlas-Computertomographie-Bilddaten als Dual-Energy Atlas-Computertomographie-Bilddaten ausgebildet sind, wobei für das atlas-basierte Verfahren geeignete monoenergetische Atlas-Computertomographie-Bilddaten aus den Dual-Energy Atlas-Computertomographie-Bilddaten abgeleitet werden.

Dual-Energy Computertomographie-Bilddaten sind dabei dem Fachmann bekannt, so dass hierbei nicht genauer auf diese eingegangen werden soll. Die Dual-Energy Atlas-Computertomographie-Bilddaten können dabei vorteilhafterweise eine zusätzliche Quelle zum Extrahieren von Gewebeinformationen darstellen. Für die jeweilige Anforderung kann dann für das atlas-basierte Verfahren ein geeigneter Energie-Level bestimmt werden, auf welchem die monoenergetischen Atlas-Computertomographie-Bilddaten aus den Dual-Energy Atlas-Computertomographie-Bilddaten bestimmt werden. Derart kann ein verbessertes Zuordnen der Atlas-Computertomographie-Bilddaten zu den Computertomographie-Bilddaten in dem atlas-basierten Verfahren erfolgen.

Eine Ausführungsform sieht vor, dass die Computertomographie-Bilddaten zusammen mit den den Computertomographie-Bilddaten zugeordneten Magnetresonanz-Bilddaten zur Unterstützung der Planung der Bestrahlung einer kompletten Organstruktur des Patienten bereitgestellt werden.

Diesem Vorgehen liegt die Überlegung zugrunde, dass die den Computertomographie-Bilddaten zugeordneten Magnetresonanz-Bilddaten gerade in Fällen der Bestrahlung der kompletten Organstruktur wertvolle Zusatzinformationen bei der Planung der Bestrahlung liefern können. So können die den Computertomographie-Bilddaten zugeordneten Magnetresonanz-Bilddaten beispielsweise besonders wertvolle Zusatzinformationen bei der Planung der Bestrahlung der gesamten Prostata, der gesamten Blase, des gesamten Rektums oder der gesamten Lunge des Patienten eingesetzt werden. Gerade für eine genauere Konturierung bzw. Segmentierung der gesamten Prostata kann das vorgeschlagene Vorgehen besonders vorteilhaft eingesetzt werden. Ein Einsatz des vorgeschlagenen Vorgehens bei der Planung der Bestrahlung von Organteilen, wie beispielsweise eines Teils des Gehirns des Patienten oder einer fokussierten Teilbestrahlung der Prostata, ist in Ausnahmefällen ebenfalls denkbar.

Ein weiteres erfindungsgemäßes Verfahren zur Unterstützung einer Planung einer Bestrahlung des Patienten umfasst folgende Verfahrensschritte:
- Erfassen von Computertomographie-Bilddaten des Patienten, welche von dem Patienten mittels eines Computertomographiegeräts akquiriert worden sind,
- Bereitstellen eines künstlichen neuronalen Netzes, welches zu einer Segmentierung einer Organstruktur basierend auf korrespondierenden Trainings-Magnetresonanz-Bilddaten und Trainings-Computertomographie-Bilddaten trainiert worden ist,
- Segmentieren der Organstruktur in den Computertomographie-Bilddaten unter Anwendung des trainierten künstlichen neuronalen Netzes auf einen Bildinhalt der Computertomographie-Bilddaten und
- Bereitstellen der Computertomographie-Bilddaten zusammen mit der segmentierten Organstruktur zur Unterstützung der Planung der Bestrahlung des Patienten.

Bezüglich des Erfassens der Computertomographie-Bilddaten wird auf die Beschreibung in einem der vorhergehenden Absätze verwiesen.

Das künstliche neuronale Netz wird für die Segmentierung der Organstruktur insbesondere aus einer Datenbank geladen. Dabei wird insbesondere ein bereits trainiertes künstliches neuronales Netz bereitgestellt. Ein künstliches neuronales Netz (KNN, englisch artificial neural network - ANN) ist insbesondere ein in einem Rechenprogramm nachgebildetes Netz aus künstlichen Neuronen. Das künstliche neuronale Netz basiert dabei typischerweise auf einer Vernetzung von mehreren künstlichen Neuronen. Die künstlichen Neuronen sind dabei typischerweise auf verschiedenen Schichten (layers) angeordnet. Üblicherweise umfasst das künstliche neuronale Netz eine Eingangsschicht und eine Ausgabeschicht (output layer), deren Neuronenausgabe als einzige des künstlichen neuronalen Netzes sichtbar wird. Zwischen der Eingangsschicht und der Ausgabeschicht liegende Schichten werden typischerweise als verdeckte Schichten (hidden layer) bezeichnet. Typischerweise wird zunächst eine Architektur und/oder Topologie eines künstlichen neuronalen Netzes initiiert und dann in einer Trainingsphase für eine spezielle Aufgabe oder für mehrere trainiert. Das Training des künstlichen neuronalen Netzes umfasst dabei typischerweise eine Veränderung einer Gewichtung einer Verbindung zwischen zwei künstlichen Neuronen des künstlichen neuronalen Netzes. Das Training des künstlichen neuronalen Netzes kann auch eine Entwicklung von neuen Verbindungen zwischen künstlichen Neuronen, ein Löschen von bestehenden Verbindungen zwischen künstlichen Neuronen, ein Anpassen von Schwellwerten der künstlichen Neuronen und/oder ein Hinzufügen oder ein Löschen von künstlichen Neuronen umfassen. Zwei unterschiedliche trainierte künstliche neuronale Netze können so, obwohl sie beispielsweise die gleiche Architektur und/oder Topologie aufweisen, unterschiedliche Aufgaben durchführen.

Es wird nun vorgeschlagen, dass ein derart trainiertes künstliches neuronales Netz für das Segmentieren der Organstruktur gewählt wird, dass es eine Segmentierung der Organstruktur ermöglicht. Das trainierte künstliche neuronale Netz kann dabei für eine spezielle Trainingsaufgabe trainiert sein, beispielsweise lediglich zur Segmentierung der Organstruktur in den Computertomographie-Bilddaten geeignet sein. Es ist denkbar, dass in der Praxis verschiedene künstliche neuronale Netze nebeneinander aufgesetzt werden, welche Segmentierungen von unterschiedlichen Organstrukturen durchführen

Im vorliegenden Verfahren wird insbesondere ein bereits trainiertes künstliches neuronales Netz für die Segmentierung der Organstruktur in den Computertomographie-Bilddaten bereitgestellt. Das Training des künstlichen neuronalen Netzes kann dabei mittels der Trainings-Magnetresonanz-Bilddaten und der Trainings-Computertomographie-Bilddaten durchgeführt worden sein. Die Trainings-Magnetresonanz-Bilddaten und Trainings-Computertomographie-Bilddaten können analog wie die Magnetresonanz-Bilddaten und Atlas-Computertomographie-Bilddaten des anderen erfindungsgemäßen Verfahrens ausgebildet sein, so dass hierbei auf die entsprechenden vorhergehenden Absätze der Beschreibung verwiesen wird.

Das Segmentieren der Organstruktur der Computertomographie-Bilddaten erfolgt insbesondere dadurch, dass das trainierte künstliche neuronale Netz ausschließlich auf den Bildinhalt der Computertomographie-Bilddaten angewandt wird. Es müssen vorteilhafterweise keine weiteren Bilddaten des Patienten für das Segmentieren der Organstruktur akquiriert werden. Der Bildinhalt der Computertomographie-Bilddaten kann derart als Eingabeinformation in das trainierte künstliche neuronale Netz eingespeist werden. Das künstliche neuronale Netz kann dann als Ausgabe, insbesondere als Ausgabe der künstlichen Neuronen der Ausgabeschicht, diejenigen Voxel in den Computertomographie-Bilddaten kennzeichnen, welche einen Teil der zu segmentierenden Organstruktur darstellen. Derart kann das trainierte künstliche neuronale Netz besonders vorteilhaft ausschließlich basierend auf dem Bildinhalt der Computertomographie-Bilddaten die Organstruktur in den Computertomographie-Bilddaten segmentieren.

Diesem Vorgehen liegt die Überlegung zugrunde, dass das künstliche neuronale Netz die Organstruktur ausschließlich basierend auf dem Bildinhalt der Computertomographie-Bilddaten segmentieren kann, da es Erfahrung bei der Segmentierung der Organstruktur in Bilddaten von verschiedenen Modalitäten gesammelt hat. So ist das künstliche neuronale Netz basierend auf korrespondierenden Trainings-Magnetresonanz-Bilddaten und Trainings-Computertomographie-Bilddaten trainiert worden. In das Training des künstlichen neuronalen Netzes sind dabei vorteilhafterweise auch Zusatzinformationen aus einer Magnetresonanztomographie eingeflossen. Geeignete Zusatzinformationen wurden dabei bereits ausführlich in einem der vorhergehenden Absätze beschrieben. Das Training hat dem künstlichen neuronalen Netz vorteilhafterweise ermöglicht, Zusammenhänge zwischen den Trainings-Magnetresonanz-Bilddaten und Trainings-Computertomographie-Bilddaten herzustellen. Diese abgespeicherten Zusammenhänge können dann besonders vorteilhaft dem künstlichen neuronalen Netz ermöglichen, die Segmentierung der Organstruktur ausschließlich auf Grundlage des Bildinhalts der Computertomographie-Bilddaten durchzuführen.

Die Computertomographie-Bilddaten können dann geeignet zusammen mit der segmentierten Organstruktur für die Unterstützung der Planung der Bestrahlung des Patienten bereitgestellt werden. So ist, wie in einem der vorhergehenden Absätze noch genauer beschrieben, beispielsweise eine Anzeige und/oder ein Abspeichern und/oder ein Übertragen zur Weiterverarbeitung der Computertomographie-Bilddaten zusammen mit der segmentierten Organstruktur für die Unterstützung der Planung der Bestrahlung des Patienten denkbar.

Dieses Verfahren lässt sich besonders vorteilhaft zur Segmentierung einer Organstruktur einsetzen, welche alleine unter Verwendung der Computertomographie-Bilddaten des Patienten schwierig zu segmentieren ist, beispielsweise da in den Computertomographie-Bilddaten die Organstruktur schwierig von ihrer Umgebung zu unterscheiden ist. Die Segmentierung der Organstruktur kann mittels dieses Vorgehens vorteilhafterweise ermöglicht werden, ohne dass eine zusätzliche Akquisition von Magnetresonanz-Bilddaten des Patienten mittels eines Magnetresonanzgeräts nötig ist. Besonders vorteilhaft ist dieses Verfahren zur Segmentierung der Prostata für die Unterstützung der Planung der Bestrahlung der Prostata denkbar. Selbstverständlich können mittels dieses Vorgehens auch Risikoorgane für die Unterstützung der Planung der Bestrahlung segmentieret werden.

Die erfindungsgemäße Recheneinheit umfasst zumindest ein Rechenmodul, wobei die Recheneinheit zum Ausführen eines erfindungsgemäßen Verfahrens ausgebildet ist.

So ist insbesondere die Recheneinheit dazu ausgebildet, computerlesbare Instruktionen auszuführen, um das erfindungsgemäße Verfahren auszuführen. Insbesondere umfasst die Recheneinheit eine Speichereinheit, wobei auf der Speichereinheit computerlesbare Informationen gespeichert sind, wobei die Recheneinheit dazu ausgebildet ist, die computerlesbaren Informationen von der Speichereinheit zu laden und die computerlesbaren Informationen auszuführen, um ein erfindungsgemäßes Verfahren auszuführen.

Derart ist die Recheneinheit dazu ausgebildet, ein Verfahren zur Unterstützung einer Planung einer Bestrahlung eines Patienten auszuführen. Dafür kann die Recheneinheit eine Bilddatenerfassungseinheit umfassen, welche zum Erfassen von Computertomographie-Bilddaten des Patienten, welche von dem Patienten mittels eines Computertomographiegeräts akquiriert worden sind, ausgebildet ist. Die Recheneinheit kann dafür weiterhin eine Zuordnungseinheit aufweisen, welche zum Zuordnen von Magnetresonanz-Bilddaten zu den Computertomographie-Bilddaten unter Verwendung der Computertomographie-Bilddaten, wobei die Magnetresonanz-Bilddaten von zumindest einem von dem Patienten verschiedenen Untersuchungsobjekt mittels eines Magnetresonanzgeräts akquiriert worden sind, ausgebildet ist. Schließlich kann die Recheneinheit eine Bereitstellungseinheit aufweisen, welche zum Bereitstellen der Computertomographie-Bilddaten zusammen mit den den Computertomographie-Bilddaten zugeordneten Magnetresonanz-Bilddaten zur Unterstützung der Planung der Bestrahlung des Patienten ausgebildet ist.

Wenn die Recheneinheit zum Durchführen des weiteren erfindungsgemäßen Verfahrens ausgebildet ist, wird die Recheneinheit insbesondere ebenfalls die Bilddatenerfassungseinheit aufweisen. Die Bereitstellungseinheit ist jedoch dann insbesondere zum Bereitstellen der Computertomographie-Bilddaten zusammen mit der segmentierten Organstruktur zur Unterstützung der Planung der Bestrahlung des Patienten ausgebildet. Weiterhin kann diese Recheneinheit dann eine weitere Bereitstellungseinheit zum Bereitstellen eines künstlichen neuronalen Netzes, welches zu einer Segmentierung einer Organstruktur basierend auf korrespondierenden Trainings-Magnetresonanz-Bilddaten und Trainings-Computertomographie-Bilddaten trainiert worden ist, umfassen. Die Recheneinheit kann dann auch eine Segmentierungseinheit zum Segmentieren der Organstruktur in den Computertomographie-Bilddaten des Patienten unter Anwendung des trainierten künstlichen neuronalen Netzes auf einen Bildinhalt der erfassten Computertomographie-Bilddaten umfassen.

Die Komponenten der Recheneinheit können zum überwiegenden Teil in Form von Softwarekomponenten ausgebildet sein. Grundsätzlich können diese Komponenten aber auch zum Teil, insbesondere wenn es um besonders schnelle Berechnungen geht, in Form von softwareunterstützten Hardwarekomponenten, beispielsweise FPGAs oder dergleichen, realisiert sein. Ebenso können die benötigten Schnittstellen, beispielsweise wenn es nur um eine Übernahme von Daten aus anderen Softwarekomponenten geht, als Softwareschnittstellen ausgebildet sein. Sie können aber auch als hardwaremäßig aufgebaute Schnittstellen ausgebildet sein, die durch geeignete Software angesteuert werden. Selbstverständlich ist es auch denkbar, dass mehrere der genannten Komponenten in Form einer einzelnen Softwarekomponente bzw. softwareunterstützter Hardwarekomponente zusammengefasst realisiert sind.

Das erfindungsgemäße Computerprogrammprodukt ist direkt in einen Speicher einer programmierbaren Recheneinheit ladbar und weist Programmcode-Mittel auf, um ein erfindungsgemäßes Verfahren auszuführen, wenn das Computerprogrammprodukt in der Recheneinheit ausgeführt wird. Das Computerprogrammprodukt kann ein Computerprogramm sein oder ein Computerprogramm umfassen. Dadurch kann das erfindungsgemäße Verfahren schnell, identisch wiederholbar und robust ausgeführt werden. Das Computerprogrammprodukt ist so konfiguriert, dass es mittels der Recheneinheit die erfindungsgemäßen Verfahrensschritte ausführen kann. Die Recheneinheit muss dabei jeweils die Voraussetzungen wie beispielsweise einen entsprechenden Arbeitsspeicher, eine entsprechende Grafikkarte oder eine entsprechende Logikeinheit aufweisen, so dass die jeweiligen Verfahrensschritte effizient ausgeführt werden können. Das Computerprogrammprodukt ist beispielsweise auf einem computerlesbaren Medium gespeichert oder auf einem Netzwerk oder Server hinterlegt, von wo es in den Prozessor einer lokalen Recheneinheit geladen werden kann, der mit direkt verbunden oder als Teil ausgebildet sein kann. Weiterhin können Steuerinformationen des Computerprogrammprodukts auf einem elektronisch lesbaren Datenträger gespeichert sein. Die Steuerinformationen des elektronisch lesbaren Datenträgers können derart ausgestaltet sein, dass sie bei Verwendung des Datenträgers in einer Recheneinheit ein erfindungsgemäßes Verfahren ausführen. So kann das Computerprogrammprodukt auch den elektronisch lesbaren Datenträger darstellen. Beispiele für elektronisch lesbare Datenträger sind eine DVD, ein Magnetband, eine Festplatte oder ein USB-Stick, auf welchem elektronisch lesbare Steuerinformationen, insbesondere Software (vgl. oben), gespeichert ist. Wenn diese Steuerinformationen (Software) von dem Datenträger gelesen und in eine Steuerung und/oder Recheneinheit gespeichert werden, können alle erfindungsgemäßen Ausführungsformen der vorab beschriebenen Verfahren durchgeführt werden. So kann die Erfindung auch von dem besagten computerlesbaren Medium und/oder dem besagten elektronisch lesbaren Datenträger ausgehen.

Die Vorteile der erfindungsgemäßen Computerprogrammprodukts und der erfindungsgemäßen Recheneinheit entsprechen im Wesentlichen den Vorteilen des erfindungsgemäßen Verfahrens bzw. des weiteren erfindungsgemäßen Verfahrens, welche vorab im Detail ausgeführt sind. Hierbei erwähnte Merkmale, Vorteile oder alternative Ausführungsformen sind ebenso auch auf die anderen beanspruchten Gegenstände zu übertragen und umgekehrt. Mit anderen Worten können die gegenständlichen Ansprüche auch mit den Merkmalen, die in Zusammenhang mit einem Verfahren beschrieben oder beansprucht sind, weitergebildet sein. Die entsprechenden funktionalen Merkmale des Verfahrens werden dabei durch entsprechende gegenständliche Module, insbesondere durch Hardware-Module, ausgebildet.

Im Folgenden wird die Erfindung anhand der in den Figuren dargestellten Ausführungsbeispiele näher beschrieben und erläutert.

Es zeigen:
Fig. 1 eine erfindungsgemäße Recheneinheit zum Ausführen eines Verfahrens zur Unterstützung einer Planung einer Bestrahlung eines Patienten,
Fig. 2 eine erste Ausführungsform eines Verfahrens zur Unterstützung einer Planung einer Bestrahlung eines Patienten,
Fig. 3 eine zweite Ausführungsform eines Verfahrens zur Unterstützung einer Planung einer Bestrahlung eines Patienten,
Fig. 4 eine Ausführungsform eines weiteren Verfahrens zur Unterstützung einer Planung einer Bestrahlung eines Patienten, welches ein künstliches neuronales Netz einsetzt, und
Fig. 5 ein Computertomographiegerät zum Aufnehmen der Computertomographie-Bilddaten des Patienten.

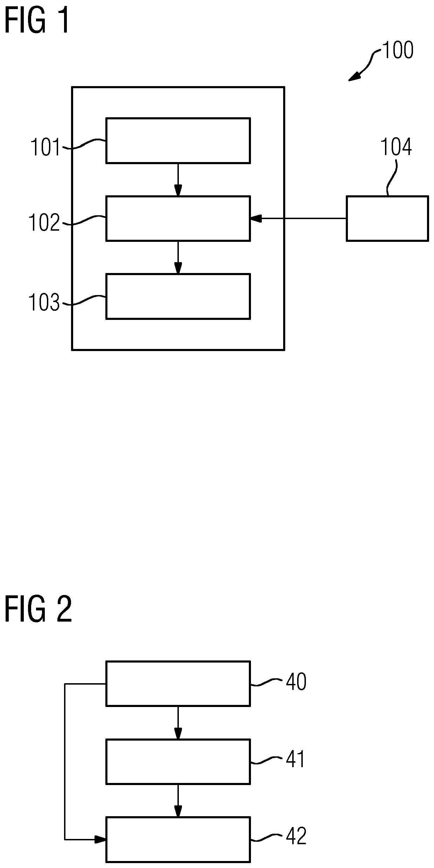

**Fig. 1** zeigt eine erfindungsgemäße Recheneinheit 100 zum Ausführen eines Verfahrens zur Unterstützung einer Planung einer Bestrahlung eines Patienten.

Die dargestellte Recheneinheit 100 umfasst mehrere Rechenmodule 101, 102, 103, mittels welcher es zum Ausführen des erfindungsgemäßen Verfahrens ausgebildet ist. So umfasst die Recheneinheit 100 eine Erfassungseinheit 101 zum Erfassen von Computertomographie-Bilddaten des Patienten. Weiterhin umfasst die Recheneinheit 100 eine Bereitstellungseinheit 103 zum Bereitstellen der Computertomographie-Bilddaten zusammen mit Zusatzdaten. Die Zusatzdaten können dabei den Computertomographie-Bilddaten zugeordnete Magnetresonanz-Bilddaten oder eine segmentierte Organstruktur sein. Die Bereitstellungseinheit 103 kann dabei vorteilhafterweise in einem Datenaustausch zu einer Weiterverarbeitungseinheit und/oder einer Anzeigeeinheit stehen, um vorteilhaft zu der Planung der Bestrahlung des Patienten die Computertomographie-Bilddaten mit den Zusatzdaten bereitstellen zu können.

Die Recheneinheit 100 umfasst ein weiteres Rechenmodul 102, welches zusätzliche Rechenschritte durchführen kann. So kann das weitere Rechenmodul 102 Magnetresonanz-Bilddaten zu den Computertomographie-Bilddaten zuordnen bzw. unter Verwendung eines trainierten künstlichen neuronalen Netzes eine Organstruktur segmentieren. Dazu kann das weitere Rechenmodul 102 in einem Datenaustausch zu einer Datenbank 104 stehen, auf welcher die Magnetresonanz-Bilddaten, insbesondere in einem Atlas zusammen mit Atlas-Computertomographie-Bilddaten, bzw. das trainierte künstliche neuronale Netz gespeichert sind.

Die dargestellte Recheneinheit 100 kann somit zur Ausführung von beiden beschriebenen erfindungsgemäßen Verfahren ausgebildet sein. Die Recheneinheit 100 kann alternativ auch lediglich zur Ausführung des erfindungsgemäßen Verfahrens, welches die Zuordnung der Magnetresonanz-Bilddaten zu den Computertomographie-Bilddaten vorsieht, ausgebildet sein. Alternativ kann die Recheneinheit 100 auch lediglich zur Ausführung des erfindungsgemäßen Verfahrens, welches die Segmentierung der Organstruktur unter Verwendung des trainierten künstlichen neuronalen Netzes vorsieht, ausgebildet sein.

**Fig. 2** zeigt eine erste Ausführungsform eines Verfahrens zur Unterstützung einer Planung einer Bestrahlung eines Patienten.

In einem ersten Verfahrensschritt 40 erfolgt ein Erfassen von Computertomographie-Bilddaten des Patienten mittels der Erfassungseinheit 101 der Recheneinheit 100, wobei die Computertomographie-Bilddaten von dem Patienten mittels eines Computertomographiegeräts akquiriert worden sind.

In einem weiteren Verfahrensschritt 41 erfolgt ein Zuordnen von Magnetresonanz-Bilddaten zu den Computertomographie-Bilddaten unter Verwendung der Computertomographie-Bilddaten mittels des weiteren Rechenmoduls 102 der Recheneinheit 100, wobei die Magnetresonanz-Bilddaten von zumindest einem von dem Patienten verschiedenen Untersuchungsobjekt mittels eines Magnetresonanzgeräts akquiriert worden sind.

In einem weiteren Verfahrensschritt 42 erfolgt ein Bereitstellen der Computertomographie-Bilddaten zusammen mit den den Computertomographie-Bilddaten zugeordneten Magnetresonanz-Bilddaten mittels der Bereitstellungseinheit 103 der Recheneinheit 100 zur Unterstützung der Planung der Bestrahlung des Patienten.

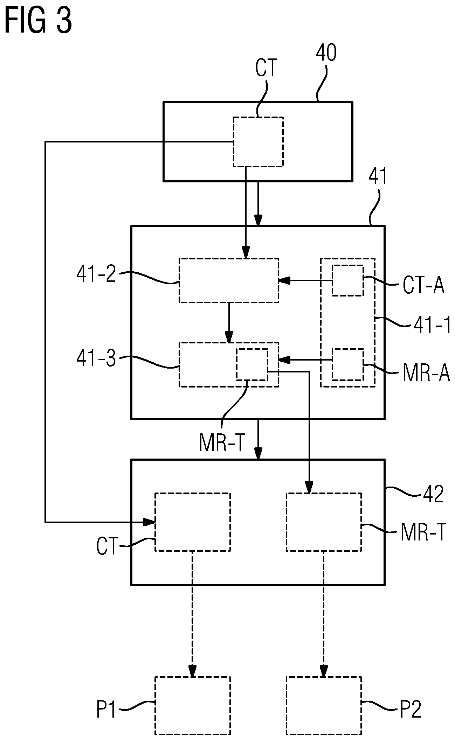

**Fig. 3** zeigt eine zweite Ausführungsform eines Verfahrens zur Unterstützung einer Planung einer Bestrahlung eines Patienten.

Die nachfolgende Beschreibung beschränkt sich im Wesentlichen auf die Unterschiede zu dem Ausführungsbeispiel in Fig. 2, wobei bezüglich gleich bleibender Verfahrensschritte auf die Beschreibung des Ausführungsbeispiels in Fig. 2 verwiesen wird. Im Wesentlichen gleich bleibende Verfahrensschritte sind grundsätzlich mit den gleichen Bezugszeichen beziffert.

Die in Fig. 3 gezeigte zweite Ausführungsform des erfindungsgemäßen Verfahrens umfasst im Wesentlichen die Verfahrensschritte 40, 41, 42 der ersten Ausführungsform des erfindungsgemäßen Verfahrens gemäß Fig. 2. Zusätzlich umfasst die in Fig. 3 gezeigte zweite Ausführungsform des erfindungsgemäßen Verfahrens zusätzliche Verfahrensschritte und/oder Unterschritte. Denkbar ist auch ein zu Fig. 3 alternativer Verfahrensablauf, welcher nur einen Teil der in Fig. 3 dargestellten zusätzlichen Verfahrensschritte und/oder Unterschritte aufweist. Selbstverständlich kann auch ein zu Fig. 3 alternativer Verfahrensablauf zusätzliche Verfahrensschritte und/oder Unterschritte aufweisen.

Die im ersten Verfahrensschritt 40 erfassten Computertomographie-Bilddaten CT werden im weiteren Verfahrensschritt 40 zusammen mit den den Computertomographie-Bilddaten CT zugeordneten Magnetresonanz-Bilddaten MR-T zur Unterstützung der Planung der Bestrahlung des Patienten bereitgestellt. Dabei werden die Computertomographie-Bilddaten CT zu einem anderen Zweck bereitgestellt werden als die den Computertomographie-Bilddaten CT zugeordneten Magnetresonanz-Bilddaten MR-T. Besonders vorteilhaft werden die Computertomographie-Bilddaten CT zusammen mit den den Computertomographie-Bilddaten CT zugeordneten Magnetresonanz-Bilddaten MR-T zur Unterstützung der Planung der Bestrahlung einer kompletten Organstruktur des Patienten bereitgestellt.

So werden die Computertomographie-Bilddaten CT zur Unterstützung der Planung der Bestrahlung zu zumindest einem ersten Zweck P1 bereitgestellt. Dieser erste Zweck P1 kann umfassen: eine Verifizierung einer korrekten Positionierung des Patienten für die Bestrahlung und/oder eine Berechnung einer Strahlendosis während der Planung der Bestrahlung.

Zur Unterstützung der Planung der Bestrahlung werden die den Computertomographie-Bilddaten CT zugeordneten Magnetresonanz-Bilddaten MR-T zu einem zweiten Zweck P2 bereitgestellt, welcher insbesondere von dem ersten Zweck P1 zumindest teilweise verschieden ist. Dieser zweite Zweck P2 kann umfassen: eine Konturierung einer Organstruktur des Patienten für die Planung der Bestrahlung und/oder eine automatische Segmentierung einer Organstruktur des Patienten für die Planung der Bestrahlung.

Gemäß Fig. 3 erfolgt das Zuordnen der Magnetresonanz-Bilddaten MR-A zu den Computertomographie-Bilddaten CT im weiteren Verfahrensschritt 41 mittels eines atlas-basierten Verfahrens. Dazu wird in einem ersten Teilschritt 41-1 des weiteren Verfahrensschritts 41 ein Atlas bereitgestellt, welcher die Magnetresonanz-Bilddaten MR-A zusammen mit korrespondierenden Atlas-Computertomographie-Bilddaten CT-A des zumindest einem von dem Patienten verschiedenen Untersuchungsobjekts umfasst.

Weiterhin erfolgt in einem zweiten Teilschritt 41-2 des weiteren Verfahrensschritts 41 ein Zuordnen der Atlas-Computertomographie-Bilddaten CT-A zu den Computertomographie-Bilddaten CT. Anschließend erfolgt in einem dritten Teilschritt 41-3 des weiteren Verfahrensschritts 41 ein Zuordnen der Magnetresonanz-Bilddaten MR-A zu den Computertomographie-Bilddaten CT unter Verwendung von Informationen, welche sich aus dem Zuordnen der Atlas-Computertomographie-Bilddaten CT-A zu den Computertomographie-Bilddaten CT ergeben. Das Ergebnis dieser Zuordnung sind dann den Computertomographie-Bilddaten CT zugeordnete Magnetresonanz-Bilddaten MR-T

Dabei kann das Zuordnen der Atlas-Computertomographie-Bilddaten CT-A zu den Computertomographie-Bilddaten CT eine Registrierung der Atlas-Computertomographie-Bilddaten CT-A auf die Computertomographie-Bilddaten CT umfassen, wobei das Zuordnen der Magnetresonanz-Bilddaten MR-A zu den Computertomographie-Bilddaten CT unter Verwendung eines bei der vorhergehenden Registrierung ermittelten Deformationsfelds erfolgt. Alternativ kann das Zuordnen der Atlas-Computertomographie-Bilddaten CT-A zu den Computertomographie-Bilddaten CT mit einer patch-basierten Methode erfolgen, wobei das Zuordnen der Magnetresonanz-Bilddaten MR-A zu den Computertomographie-Bilddaten CT unter Verwendung von Informationen erfolgt, welche bei der patch-basierten Methode gewonnen werden. Es ist auch denkbar, dass die Atlas-Computertomographie-Bilddaten CT-A als Dual-Energy Atlas-Computertomographie-Bilddaten ausgebildet sind, wobei für das atlas-basierte Verfahren geeignete monoenergetische Atlas-Computertomographie-Bilddaten aus den Dual-Energy Atlas-Computertomographie-Bilddaten abgeleitet werden.

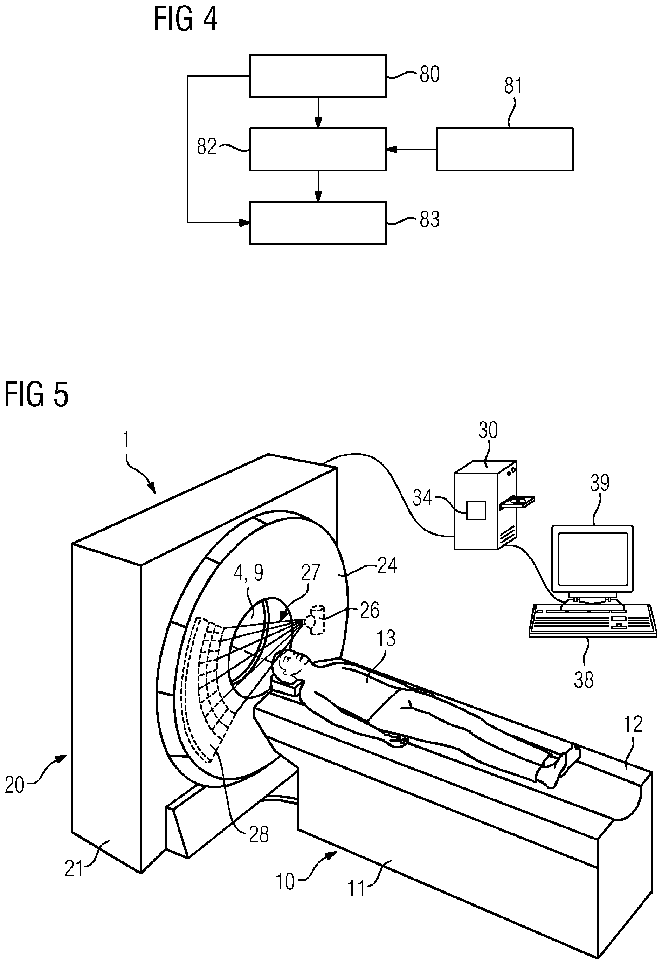

**Fig. 4** zeigt eine Ausführungsform eines weiteren Verfahrens zur Unterstützung einer Planung einer Bestrahlung eines Patienten, welches ein künstliches neuronales Netz einsetzt.

In einem ersten Verfahrensschritt 80 erfolgt ein Erfassen von Computertomographie-Bilddaten des Patienten mittels der Erfassungseinheit 101 der Recheneinheit 100, wobei die Computertomographie-Bilddaten von dem Patienten mittels eines Computertomographiegeräts akquiriert worden sind.

In einem weiteren Verfahrensschritt 81 erfolgt ein Bereitstellen eines künstlichen neuronalen Netzes mittels des weiteren Rechenmoduls 102 der Recheneinheit 100, welches auf die Datenbank 104 zugreift. Das künstliche neuronale Netz ist zu einer Segmentierung einer Organstruktur basierend auf korrespondierenden Trainings-Magnetresonanz-Bilddaten und Trainings-Computertomographie-Bilddaten trainiert worden.

In einem weiteren Verfahrensschritt 82 erfolgt ein Segmentieren der Organstruktur in den Computertomographie-Bilddaten mittels des weiteren Rechenmoduls 102 der Recheneinheit 100 unter Anwendung des trainierten künstlichen neuronalen Netzes auf einen Bildinhalt der Computertomographie-Bilddaten.

In einem weiteren Verfahrensschritt 83 erfolgt ein Bereitstellen der Computertomographie-Bilddaten zusammen mit der segmentierten Organstruktur mittels der Bereitstellungseinheit 103 der Recheneinheit 100 zur Unterstützung der Planung der Bestrahlung des Patienten.

Die in Fig. 2-4 dargestellten Verfahrensschritte des erfindungsgemäßen Verfahrens werden von der Recheneinheit ausgeführt. Hierzu umfasst die Recheneinheit erforderliche Software und/oder Computerprogramme, die in einer Speichereinheit der Recheneinheit gespeichert sind. Die Software und/oder Computerprogramme umfassen Programmmittel, die dazu ausgelegt sind, das erfindungsgemäße Verfahren auszuführen, wenn das Computerprogramm und/oder die Software in der Recheneinheit mittels einer Prozessoreinheit der Recheneinheit ausgeführt wird.

**Fig. 5** zeigt ein Computertomographiegerät 1 zum Akquirieren der Computertomographie-Bilddaten des Patienten. Die mittels des Computertomographiegeräts 1 akquirierten Computertomographie-Bilddaten können dann in dem Verfahren zur Unterstützung der Planung der Bestrahlung des Patienten verwendet werden. Dafür können die Computertomographie-Bilddaten von dem Computertomographiegerät 1 an die erfindungsgemäße Recheneinheit 100 übertragen werden, welche die Computertomographie-Bilddaten mittels der Erfassungseinheit 101 erfassen und anschließend weiterverarbeiten kann.

Das Computertomographiegerät 1 weist eine Gantry 20, eine tunnelförmige Öffnung 9, eine Patientenlagerungsvorrichtung 10 und eine Steuerungsvorrichtung 30 auf. Die Gantry 20 weist einen stationären Tragrahmen 21 und einen Rotor 24 auf. Der Rotor 24 ist mittels einer Drehlagerungsvorrichtung um eine Rotationsachse relativ zu dem stationären Tragrahmen 21 drehbar an dem stationären Tragrahmen 21 angeordnet. In die tunnelförmige Öffnung 9 ist ein Patient 13 einführbar. In der tunnelförmigen Öffnung 9 befindet sich ein Akquisitionsbereich 4. In dem Akquisitionsbereich 4 ist ein abzubildender Bereich des Patienten 13 derart positionierbar, dass eine elektromagnetische Strahlung 27 von einer Strahlungsquelle 26 zu dem abzubildenden Bereich und nach einer Wechselwirkung mit dem abzubildenden Bereich zu einem Strahlungsdetektor 28 gelangen kann. Die Patientenlagerungsvorrichtung 10 weist einen Lagerungstisch 11 und eine Transferplatte 12 zur Lagerung des Patienten 13 auf. Die Transferplatte 12 ist derart relativ zu dem Lagerungstisch 11 bewegbar an dem Lagerungstisch 11 angeordnet, dass die Transferplatte 12 in einer Längsrichtung der Transferplatte 12 in den Akquisitionsbereich 4 einführbar ist.

Das Computertomographiegerät 1 ist zur Akquisition von Projektionsdaten basierend auf der elektromagnetischen Strahlung 27 ausgebildet. Das Computertomographiegerät 1 umfasst eine Projektionsdaten-Akquisitionseinheit mit der Strahlungsquelle 26, insbesondere einer Röntgenquelle, und dem Detektor 28, beispielsweise einem Röntgendetektor, insbesondere einem energieauflösenden Röntgendetektor. Die Strahlungsquelle 26 ist an dem Rotor 24 angeordnet und zur Emission einer Strahlung 27, insbesondere einer Röntgenstrahlung, mit Strahlungsquanten 27 ausgebildet. Der Detektor 28 ist an dem Rotor 24 angeordnet und zur Detektion der Strahlungsquanten 27 ausgebildet. Die Strahlungsquanten 27 können von der Strahlungsquelle 26 zu dem abzubildenden Bereich des Patienten 13 gelangen und nach einer Wechselwirkung mit dem abzubildenden Bereich auf den Detektor 28 auftreffen. Auf diese Weise können mittels der Erfassungseinheit Projektionsdaten des abzubildenden Bereichs erfasst werden.

Eine Steuerungsvorrichtung 30 ist zum Empfangen der von der Erfassungseinheit erfassten Projektionsdaten ausgebildet. Die Steuerungsvorrichtung 30 ist zum Steuern des Computertomographiegeräts 1 ausgebildet. Die Steuerungsvorrichtung 30 weist eine Bildrekonstruktionseinrichtung 34 auf. Mittels der Bildrekonstruktionseinrichtung 34 können basierend auf den Projektionsdaten Computertomographie-Bilddaten rekonstruiert werden.

Das Computertomographiegerät 1 weist eine Eingabeeinheit 38 und eine Anzeigeeinheit 39 auf, welche jeweils mit der Steuerungsvorrichtung 30 verbunden sind. Die Eingabeeinheit 38 ist zum Eingeben von Steuerungs-Informationen, z. B. Bildrekonstruktionsparametern und/oder Untersuchungsparametern, ausgebildet. Die Anzeigeeinheit 39 ist insbesondere zum Anzeigen der Computertomographie-Bilddaten ausgebildet.

Obwohl die Erfindung im Detail durch die bevorzugten Ausführungsbeispiele näher illustriert und beschrieben wurde, so ist die Erfindung dennoch nicht durch die offenbarten Beispiele eingeschränkt und andere Variationen können vom Fachmann hieraus abgeleitet werden, ohne den Schutzumfang der Erfindung zu verlassen.

## Patentansprüche

1. Verfahren zur Unterstützung einer Planung einer Bestrahlung eines Patienten (13), umfassend folgende Verfahrensschritte:
- Erfassen von Computertomographie-Bilddaten des Patienten (13), welche von dem Patienten (13) mittels eines Computertomographiegeräts (1) akquiriert worden sind,
- Zuordnen von Magnetresonanz-Bilddaten zu den Computertomographie-Bilddaten unter Verwendung der Computertomographie-Bilddaten,
- Bereitstellen der Computertomographie-Bilddaten zusammen mit den den Computertomographie-Bilddaten zugeordneten Magnetresonanz-Bilddaten zur Unterstützung der Planung der Bestrahlung des Patienten (13),
**dadurch gekennzeichnet, dass** die Magnetresonanz-Bilddaten von zumindest einem von dem Patienten (13) verschiedenen Untersuchungsobjekt mittels eines Magnetresonanzgeräts akquiriert worden sind.

2. Verfahren nach Anspruch 1, wobei zur Unterstützung der Planung der Bestrahlung die Computertomographie-Bilddaten zu einem anderen Zweck bereitgestellt werden als die den Computertomographie-Bilddaten zugeordneten Magnetresonanz-Bilddaten.

3. Verfahren nach Anspruch 2, wobei zur Unterstützung der Planung der Bestrahlung die Computertomographie-Bilddaten zu zumindest einem der folgenden Zwecke bereitgestellt werden:
- eine Verifizierung einer korrekten Positionierung des Patienten (13) für die Bestrahlung und
- eine Berechnung einer Strahlendosis während der Planung der Bestrahlung.

4. Verfahren nach einem der Ansprüche 2-3, wobei zur Unterstützung der Planung der Bestrahlung die den Computertomographie-Bilddaten zugeordneten Magnetresonanz-Bilddaten zu zumindest einem der folgenden Zwecke bereitgestellt werden:
- eine Konturierung einer Organstruktur des Patienten (13) für die Planung der Bestrahlung und
- eine automatische Segmentierung einer Organstruktur des Patienten (13) für die Planung der Bestrahlung.

5. Verfahren nach einem der vorhergehenden Ansprüche, wobei das Zuordnen der Magnetresonanz-Bilddaten zu den Computertomographie-Bilddaten mittels eines atlas-basierten Verfahrens erfolgt, welches folgende Schritte umfasst:
- Bereitstellen eines Atlas, welcher die Magnetresonanz-Bilddaten zusammen mit korrespondierenden Atlas-Computertomographie-Bilddaten des zumindest einem von dem Patienten (13) verschiedenen Untersuchungsobjekts umfasst,
- Zuordnen der Atlas-Computertomographie-Bilddaten zu den Computertomographie-Bilddaten,
- Zuordnen der Magnetresonanz-Bilddaten zu den Computertomographie-Bilddaten unter Verwendung von Informationen, welche sich aus dem Zuordnen der Atlas-Computertomographie-Bilddaten zu den Computertomographie-Bilddaten ergeben.

6. Verfahren nach Anspruch 5, wobei das Zuordnen der Atlas-Computertomographie-Bilddaten zu den Computertomographie-Bilddaten eine Registrierung der Atlas-Computertomographie-Bilddaten auf die Computertomographie-Bilddaten umfasst, wobei das Zuordnen der Magnetresonanz-Bilddaten zu den Computertomographie-Bilddaten unter Verwendung eines bei der vorhergehenden Registrierung ermittelten Deformationsfelds erfolgt.

7. Verfahren nach Anspruch 5, wobei das Zuordnen der Atlas-Computertomographie-Bilddaten zu den Computertomographie-Bilddaten mit einer patch-basierten Methode erfolgt, wobei das Zuordnen der Magnetresonanz-Bilddaten zu den Computertomographie-Bilddaten unter Verwendung von Informationen erfolgt, welche bei der patch-basierten Methode gewonnen werden.

8. Verfahren nach einem der Ansprüche 5-7, wobei die Atlas-Computertomographie-Bilddaten als Dual-Energy Atlas-Computertomographie-Bilddaten ausgebildet sind, wobei für das atlas-basierte Verfahren geeignete monoenergetische Atlas-Computertomographie-Bilddaten aus den Dual-Energy Atlas-Computertomographie-Bilddaten abgeleitet werden.

9. Verfahren nach einem der vorhergehenden Ansprüche, wobei die Computertomographie-Bilddaten zusammen mit den den Computertomographie-Bilddaten zugeordneten Magnetresonanz-Bilddaten zur Unterstützung der Planung der Bestrahlung einer kompletten Organstruktur des Patienten (13) bereitgestellt werden.

10. Recheneinheit (100), umfassend zumindest ein Rechenmodul (101, 102, 103), wobei die Recheneinheit (100) zum Ausführen eines Verfahrens nach einem der vorhergehenden Ansprüche ausgebildet ist.

11. Computerprogrammprodukt, welches direkt in einen Speicher einer programmierbaren Recheneinheit (100) ladbar ist, mit Programmcode-Mitteln, um ein Verfahren nach einem der Ansprüche 1-9 auszuführen, wenn das Computerprogrammprodukt in der Recheneinheit (100) ausgeführt wird.

## Claims

1. Method for supporting radiation treatment planning for a patient (13), including the following process steps:
- acquiring computer tomography image data from the patient (13), which has been acquired from the patient (13) using a computer tomography device (1),
- allocating magnetic resonance image data to the computer tomography image data using the computer tomography image data,
- providing the computer tomography image data together with the magnetic resonance image data allocated to the computer tomography image data to support the radiation treatment planning for the patient (13),
**characterised in that** the magnetic resonance image data has been acquired from at least one examination subject that is different from the patient (13) using a magnetic resonance unit.

2. Method according to claim 1, wherein to support the planning of radiation treatment, the computer tomography image data is provided for a different purpose than the magnetic resonance image data allocated to the computer tomography image data.

3. Method according to claim 2, wherein to support the radiation treatment planning, the computer tomography image data is provided for at least one of the following purposes:
- verification of the correct positioning of the patient (13) for radiation treatment and
- calculation of an X-ray dose during the radiation treatment planning.

4. Method according to one of claims 2-3, wherein to support the radiation treatment planning, the magnetic resonance image data allocated to the computer tomography image data is provided for at least one of the following purposes:
- a contouring of an organ structure of the patient (13) for the radiation treatment planning and
- an automatic segmentation of an organ structure of the patient (13) for the radiation treatment planning.

5. Method according to one of the preceding claims, wherein the allocation of the magnetic resonance image data to the computer tomography image data ensues using an atlas-based method, which includes the following steps:
- providing an atlas, which includes the magnetic resonance image data together with corresponding atlas-based computer tomography image data for the least one examination subject that differs from the patient (13),
- allocating the atlas-based computer tomography image data to the computer tomography image data,
- allocating the magnetic resonance image data to the computer tomography image data using information which is derived from the allocation of the atlas-based computer tomography image data to the computer tomography image data.

6. Method according to claim 5, wherein the allocation of the atlas-based computer tomography image data to the computer tomography image data includes a registration of the atlas-based computer tomography image data onto the computer tomography image data, wherein the allocation of the magnetic resonance image data to the computer tomography image data ensues using a deformation field defined in the preceding registration.

7. Method according to claim 5, wherein the allocation of the atlas-based computer tomography image data to the computer tomography image data ensues with a patch-based method, wherein the allocation of the magnetic resonance image data to the computer tomography image data ensues using information obtained with the patch-based method.

8. Method according to one of claims 5-7, wherein the atlas-based computer tomography image data is designed as dual-energy atlas-based computer tomography image data, wherein single-energy atlas-based computer tomography image data suitable for the atlas-based method is derived from the dual-energy atlas-based computer tomography image data.

9. Method according to one of the preceding claims, wherein the computer tomography image data together with the magnetic resonance image data allocated to the computer tomography image data are provided to support the planning for radiation treatment of a complete organ structure of the patient (13).

10. Computation unit (100), including at least one computation module (101, 102, 103), wherein the computation unit (100) is designed to carry out a method according to one of the preceding claims.

11. Computer program product, which can be loaded directly into a memory of a programmable computation unit (100), comprising program-coding means, in order to perform a method according to one of claims 1-9 when the computer program product is run in the computation unit (100).

## Revendications

1. Procédé de facilitation de la planification de l'exposition d'un patient (13) à un rayonnement, comprenant les stades de procédé suivants :
- détection de données d'image de tomographie à ordinateur du patient (13), qui ont été acquises du patient (13) au moyen d'un appareil (1) de tomographie à ordinateur,
- association de données d'image de résonance magnétique aux données d'image de tomographie à ordinateur en utilisant les données d'image de tomographie à ordinateur,
- mise à disposition des données d'image de tomographie à ordinateur ensemble avec les données d'image de résonance magnétique associées aux données d'image de tomographie à ordinateur pour faciliter la planification de l'exposition du patient (13) à un rayonnement,
**caractérisé en ce que** l'on a acquis les données d'image de résonance magnétique au moyen d'un appareil à résonance magnétique d'au moins un objet examiné différent du patient (13).

2. Procédé suivant la revendication 1, dans lequel, pour faciliter la planification de l'exposition à du rayonnement, on se procure les données d'image de tomographie à ordinateur pour un but autre que les données d'image de résonance magnétique associées aux données d'image de tomographie à ordinateur.

3. Procédé suivant la revendication 2, dans lequel, pour faciliter la planification de l'exposition à du rayonnement, on se procure les données d'image de tomographie à ordinateur pour au moins l'un des buts suivants :
- une vérification d'un positionnement correct du patient (13) pour l'exposition à du rayonnement et
- un calcul d'une dose de rayonnement pendant la planification de l'exposition à du rayonnement.

4. Procédé suivant l'une des revendications 2 à 3, dans lequel, pour faciliter la planification de l'exposition à du rayonnement, on se procure les données d'image de résonance magnétique associées aux données d'image de tomographie à ordinateur pour au moins l'un des buts suivants :
- l'obtention du contour d'une structure d'organe du patient (13) pour la planification de l'exposition à du rayonnement et
- une segmentation automatique d'une structure d'organe du patient (13) pour la planification de l'exposition à du rayonnement.

5. Procédé suivant l'une des revendications précédentes, dans lequel l'association des données d'image de résonance magnétique aux données d'image de tomographie à ordinateur s'effectue au moyen d'un procédé reposant sur un atlas, qui comprend les stades suivants :
- on se procure un atlas, qui comprend les données d'image de résonance magnétique ensemble avec les données d'image de tomographie à ordinateur d'atlas correspondantes du au moins un objet examiné différent du patient (13),
- on associe les données d'image de tomographie à ordinateur d'Atlas aux données d'image de tomographie à ordinateur,
- on associe les données d'image de résonance magnétique aux données d'image de tomographie à ordinateur en utilisant des informations, qui proviennent de l'association des données d'image de tomographie à ordinateur d'atlas aux données d'image de tomographie à ordinateur.

6. Procédé suivant la revendication 5, dans lequel l'association des données d'image de tomographie à ordinateur d'atlas aux données d'image de tomographie à ordinateur comprend un enregistrement des données d'image de tomographie à ordinateur d'atlas sur les données d'image de tomographie à ordinateur, l'association des données d'image de résonance magnétique aux données d'image de tomographie à ordinateur s'effectuant en utilisant un champ de déformation déterminé lors de l'enregistrement précédent.

7. Procédé suivant la revendication 5, dans lequel l'association des données d'image de tomographie à ordinateur d'atlas aux données d'image de tomographie à ordinateur s'effectue par un procédé reposant sur un patch, l'association des données d'image de résonance magnétique aux données d'image de tomographie à ordinateur s'effectuant en utilisant des informations obtenues par le procédé reposant sur un patch.

8. Procédé suivant l'une des revendications 5 à 7, dans lequel les données d'image de tomographie à ordinateur d'atlas sont constituées sous la forme de données d'image de tomographie à ordinateur d'atlas à énergie dual, dans lequel, pour le procédé reposant sur un atlas, on déduit des données d'image de tomographie à ordinateur d'atlas monoénergétiques appropriées des données d'image de tomographie à ordinateur d'Atlas à énergie dual.

9. Procédé suivant l'une des revendications précédentes, dans lequel on se procure les données d'image de tomographie à ordinateur ensemble avec les données d'image de résonance magnétique associées aux données d'image de tomographie à ordinateur pour faciliter la planification de l'exposition d'une structure d'organe complète du patient (13) à du rayonnement.

10. Unité (100) d'ordinateur, comprenant au moins un module (101, 102, 103) d'ordinateur, l'unité (100) d'ordinateur étant constituée pour réaliser un procédé suivant l'une des revendications précédentes.

11. Produit de programme d'ordinateur, qui peut être chargé directement dans une mémoire d'une unité (100) d'ordinateur programmable, ayant des moyens de code de programme pour exécuter un procédé suivant l'une des revendications 1 à 9, lorsque le produit de programme d'ordinateur est réalisé dans l'unité (100) d'ordinateur.
